# EUROPÄISCHE PATENTANMELDUNG

(11) **EP 0 598 678 A1**
(43) Veröffentlichungstag der Anmeldung: **25.05.1994**
(21) Anmeldenummer: 93810725.7
(22) Anmeldetag: 14.10.1993
(51) Int. Cl.: F16N 11/08, F16N 11/04

(54) **Schmierstoffgeber**

(30) Priorität: 16.11.1992 CH 522/92
(71) Anmelder: Grossenbacher, Beat, CH-4707 Deitingen (CH)
(72) Erfinder: Grossenbacher, Beat, CH-4707 Deitingen (CH)
(74) Vertreter: Feldmann, Paul David

(57) **Zusammenfassung**

Es wird ein Schmierstoffgeber mit einem mit Schmierstoff füllbaren Behälter (2) vorgestellt, welcher mittels Federdruck Schmierstoff aus der Auslassöffnung (3) stösst. Die Schmierstoffabgabe erfolgt durch einen Timer (111) zeitlich kontrolliert und unabhängig von inneren und äusseren Bedingungen über einen im voraus bestimmbaren Zeitraum. Der Schmierstoffgeber eignet für alle Maschinen, deren Lagerungen regelmässig geschmiert werden müsse. Durch deren Einsatz wird es möglich, die Wartungsintervalle nach betrieblichen Gesichtspunkten anzusetzen.

## Beschreibung

Die Erfindung betrifft einen Schmierstoffgeber mit einem mit Schmierstoff füllbaren Behälter, welcher auf einer Seite eine Auslassöffnung aufweist und auf der anderen Seite einen über eine Feder auf einen Deckel abgestützten Kolben, welcher mittels dem Federdruck Schmierstoff aus der Auslassöffnung stösst

Auf dem Markt sind Schmierstoffgeber, welche über eine gewisse Zeit Schmierstoff an eine bestimmte Stelle abgeben, bekannt. Sie werden zum Beispiel bei Verkehrsfahrzeugen der öffentlichen Betriebe eingebaut, um die Wartungsintervalle erheblich zu verlängern. Diese Schmierstoffgeber bestehen aus einem mit Schmierstoff füllbaren Behälter, welcher auf einer Seite eine Auslassöffnung aufweist. Auf der anderen Seite ist ein Deckel und im Innern ein Kolben. Der Kolben ist über eine Feder auf den Deckel abgestützt. Der Hohlraum zwischen Kolben und Auslassöffnung wird mit Schmierstoff gefüllt. Nach der Montage wird der Kolben durch den Federdruck gegen die Auslassöffnung gedrückt, wobei laufend eine gewisse Menge Schmierstoff aus der Auslassöffnung gepresst wird. Diese Menge ist nicht konstant, sondern vom augenblicklichen Federdruck, der Temperatur und anderen äusseren Bedingungen abhängig. Die Schmierstoffgeber werden nach einer bestimmten Betriebszeit ausgewechselt, unabhängig davon, ob sie noch Schmierstoff enthalten, oder ob sie schon längere Zeit leer sind.

Neuerdings sind Schmierstoffgeber bekannt geworden, bei welchen der Kolben nicht durch eine Feder bewegt wird, sondern durch Gasdruck in einem Druckraum zwischen Deckel und Kolben. Dabei wird Wasserstoff durch eine Gaszelle in zeitlich konstanter Menge im Druckraum selbst produziert. In einer anderen Variante wird in zeitlich konstanter Menge ein Gas aus einem Gasbehälter bezogen. Durch das zunehmende Gasvolumen im Druckraum wird der Kolben in Richtung Auslassöffnung geschoben und dabei Schmierstoff ausgestossen. Damit kann die Schmierstoffabgabe entsprechend den gewünschten Wartungsintervallen gewählt werden. Es bleiben aber immer noch die Temperaturunterschiede, welche die Gasproduktion und das Gasvolumen selbst beeinflussen. Ein besonderes Problem bildet die Abdichtung der Schmierstoffgeber gegen Gasverluste. Diese unkontrollierbaren Gasverluste beeinträchtigen die Zuverlässigkeit dieser Systeme erheblich und die Schmierstoffabgabe wird unregelmässig und unzuverlässig.

Aufgabe der Erfindung ist es, einen Schmierstoffgeber obgenannter Art zu schaffen, bei welchem die Schmierstoffabgabe kontrolliert und unabhängig von inneren und äusseren Bedingungen über einen bestimmbaren Zeitraum zuverlässig und konstant erfolgt.

Diese Aufgabe wird durch die in den Patentansprüchen angegebene Erfindung gelöst.

Die Erfindung wird nachstehend im Zusammenhang mit den Zeichnungen beschrieben. Es zeigen:
- Figur 1: einen erfindungsgemässen Schmierstoffgeber im Längsschnitt im teilweise entleerten Zustand;
- Figur 2: eine andere Ausführungsform des Schmierstoffgebers im Längsschnitt im gefüllten und im entleerten Zustand;
- Figur 3: die Steuereinrichtung im Deckel des Schmierstoffgebers.

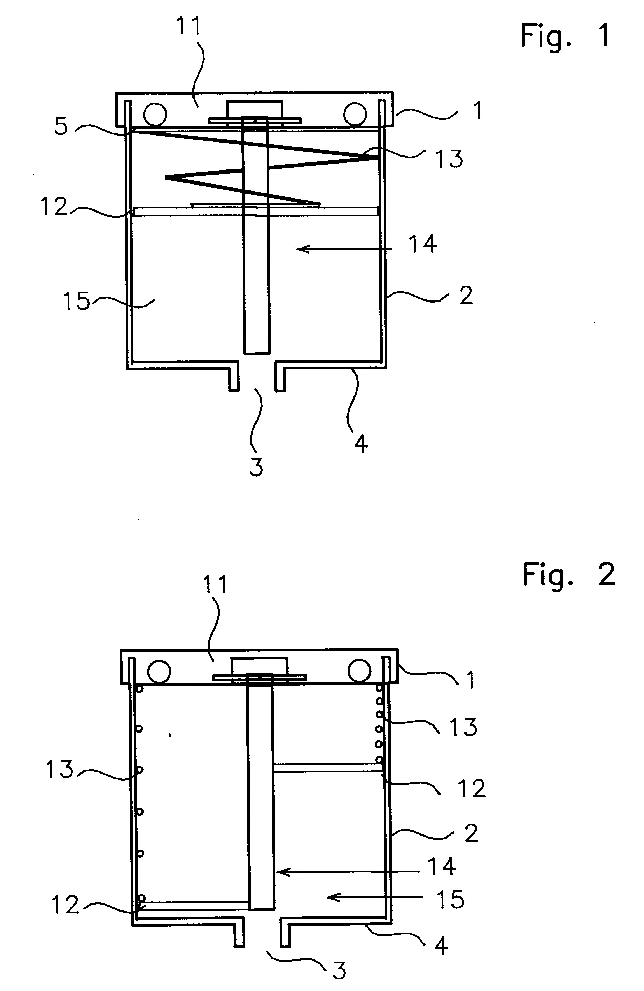

Der Schmierstoffgeber nach Figur 1 besteht aus einem mit Schmierstoff füllbaren Behälter 2, welcher auf einer Seite am Boden 4 eine Auslassöffnung 3 aufweist. Auf der anderen Seite ist der Behälter mit einem Deckel 1 fest verschlossen. Der Deckel 1 ist drehbar gegenüber einer unterhalb und parallel dazu und fest im Behälter 2 angebrachten Zwischenboden 5.

Ein Kolben 12 stützt sich über eine Feder 13 auf eine Zwischenboden 5 ab. Der Kolben 12 presst mittels dem Druck der Feder 13 Schmierstoff aus einem Vorratsraum 15, der sich zwischen Kolben 12 und Boden 4 befindet, durch die Auslassöffnung 3 an eine zu schmierende Stelle.

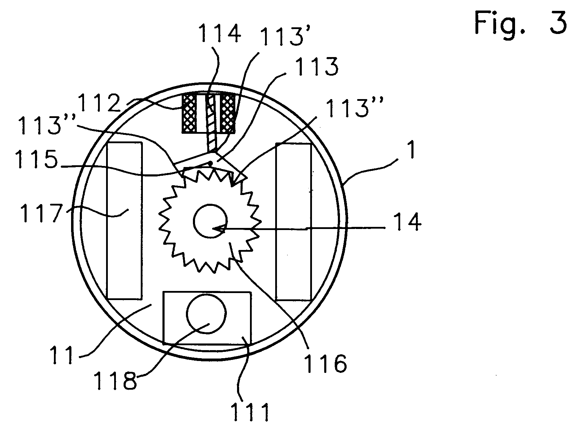

Zwischen Deckel 1 und Zwischenboden 5 befindet sich ein Zwischenraum, in dem eine Steuereinrichtung 11, welche in Figur 3 dargestellt ist, untergebracht ist. Eine Gewindestange 14 ist mit der Steuereinrichtung 11 wirkverbunden und gegenüber dem Behälter 2 drehbar in dem Zwischenboden 5 am Deckel 1 gelagert. Diese Gewindestange 14 reicht von der Steuereinrichtung 11 durch den Zwischenboden 5 in Richtung Auslassöffnung 3. Dabei durchstösst sie den Kolben 12. Der Kolben 12, welcher im Behälter 2 an dessen Innenwand gleitet, ist mittig mit einer Bohrung mit entsprechendem Gewinde versehen. Eine Feder 13 stösst den Kolben 12 von dem Zwischenboden 5 weg in Richtung Auslassöffnung 3. Die Feder 13 verhindert zudem ein Verdrehen des Kolbens 12 gegenüber dem Gehäuse 2, so dass sich der Kolben 12 nur in Längsrichtung vom Deckel 1 gegen die Auslassöffnung 3 bewegen kann. Das Gewinde der Gewindestange 14 im Innengewinde des Kolbens 12 fixiert den Kolben 12, solange die Gewindestange 14 nicht gedreht wird.

Die Steuereinrichtung 11 dreht in bestimmbaren Zeitintervallen die Gewindestange 14 um jeweils einen gewissen Winkel. Dadurch wird der Kolben 12 um einen entsprechenden Betrag in Längsrichtung verschoben. Bei dieser Drehung unterstützt die Feder 13 die Bewegung des Kolbens 12. Die Bewegung des Kolbens 12 bewirkt ein dosiertes Ausstossen von Schmierstoff aus dem Vorratsraum 15 durch die Auslassöffnung 3. Dabei wird die zum Auspressen aufzuwendende Kraft von der Feder 13 erzeugt. Die Steuereinheit 11 bestimmt mittels Drehung der Gewindestange 14 über die Steigung des Gewindes den Betrag der Kolbenverschiebung. Die Feder 13 ist in diesem Beispiel eine konische Feder, welche einen konstanten Federdruck ausübt.

Der Schmierstoffgeber ist in Figur 2 im Längsschnitt im gefüllten und im entleerten Zustand dargestellt. Er ist gleich, wie oben beschrieben, aufgebaut. Im Unterschied dazu ist die Feder 13 zylindrisch statt konisch.

Auf der einen Seite ist der Kolben 12 und die Feder 13 in der Position des vollständig entleerten Zustandes des Schmierstoffgebers dargestellt. Der Kolben 12 liegt dabei mit seiner Unterseite am Boden 4 des Behälter 2 an. Die Feder 13 ist auf die entsprechende Länge gedehnt.

Auf der anderen Seite ist der Schmierstoffgeber mit vollständig gefülltem Vorratsraum 15 dargestellt. Der Kolben 12 befindet sich in der obersten Position und die Feder 13 ist eingefedert.

In Figur 3 ist die Steuereinrichtung 11 in Ansicht von oben dargestellt. Im Deckel 1 befindet sich in der Mitte die Gewindestange 14 fest mit einem gezahnten Rad 116 verbunden Ein regulierbarer Timer 111, gespiesen durch Batterien 117, gibt in bestimmbaren Zeitintervallen elektrische Impulse an einen Elektromagneten 112 ab. Der Elektromagnet 112 zieht bei jedem erhaltenen Impuls eine Ecke 113' einer gewinkelten Wippe 113 über einen Totpunkt, welcher durch die Federwirkung einer Feder 114 auf die Ecke 113' und die relative Lage dieser Ecke 113' gegenüber dem Drehpunkt 115 der Wippe 113 und dem Elektromagnet 112 bestimmt ist. Auf diese Weise befindet sich die Wippe 113, abgesehen von der Impulsdauer, immer abwechselnd auf der einen oder anderen Seite in einem Ruhezustand. Dabei greift die Wippe 113 mit einem der beiden gezahnten Enden 113'' in die Verzahnung des gezahnten Rades 116. Die Wippe kann auch einseitig ausgeführt werden, wobei eine Feder als Rückstellkraft dient.

Die Steuervorrichtung funktioniert wie der Anker, das Ankerrad und die Hebelscheibe bei einer Uhr. Bei jedem Impuls dreht die Wippe 113 das gezahnte Rad 116 und damit die Gewindestange 14 um einen bestimmten, einer halben Zahnbreite entsprechenden, Winkel. Dies bewirkt jedesmal eine bestimmte Längsbewegung des Kolbens 12 im Behälter 2. Der Timer 111 ist mittels einem auf ihm befindlichen Potentiometer 118 steuerbar. Für die Einstellung des Timers 111 wird der Deckel 1 abgeladen. Mittels sichtbaren Markierungen auf dem Timer 111 wird ddas Potentiometer 118 damit der Schmierstoffgeber auf eine bestimmte Abgabedauer eingestellt und die Abgabe initialisiert. Während der Abgabedauer wird der Vorratsraum vollständig entleert. Dadurch ist auch die Dosierung, also die Abgabemenge pro Zeiteinheit, gegeben.
Die Abgabedauer kann beispielsweise auf ein Jahr eingestellt werden. Dann ist der Vorrat an Schmierstoff erschöpft und der Schmierstoffgeber wird ausgetauscht. Er kann nachgefüllt werden und die Batterien können bei Bedarf ausgetauscht oder nachgeladen werden. Zum Nachfüllen von Schmierstoff in den Vorratsraum ist die Gewindestange 14 mittels einer lösbaren Sperre mit dem gezahnten Rad 116 verbunden. Die hier nicht näher dargestellte lösbare Sperre ist während dem Nachfüllvorgang gelöst.
Die zeitlich geregelte Steuereinrichtung zusammen mit dem durch das Gewinde und die Drehwinkel bestimmte Kolbenbewegung garantieren eine gleichmässige, konstante und von äusseren Einflüssen unabhängige Schmierstoffabgabe.
Der Schmierstoffgeber ist überall einsetzbar, wo lange und regelmässig gemiert werden muss. Empfehlenswert ist insbesondere ein Einbau in Fahrzeuge des öffentlichen Verkehrs, Autocars, Lastwagen, Eisenbahnfahrzeuge, usw.. Durch deren Einsatz wird es möglich, die Wartungsintervalle nach betrieblichen Gesichtspunkten anzusetzen.

## Patentansprüche

1. Schmierstoffgeber mit einem mit Schmierstoff füllbaren Behälter (2), welcher auf einer Seite eine Auslassöffnung (3) aufweist und auf der anderen Seite einen über eine Feder (13) abgestützten längsverschiebbaren Kolben (12), welcher mittels dem Federdruck Schmierstoff aus der Auslassöffnung (3) stösst und mit einem Deckel (1), dadurch gekennzeichnet, dass im Behälter (2) in einem, mit diesem fest verbundenen, Zwischenboden (5), unterhalb einem Deckel (1), eine drehbar gelagerte Gewindestange (14) angeordnet ist, welche den Kolben (12) in einer Durchbohrung drehbar durchstösst und mittels dem Gewinde im Eingriff mit einem Gewinde in der Durchbohrung des Kolbens (12) ist.

2. Schmierstoffgeber nach Anspruch 1, dadurch gekennzeichnet, dass die Längsbewegung des Kolbens (12) mittels Drehen der Gewindestange (14) steuerbar ist.

3. Schmierstoffgeber nach Anspruch 2, dadurch gekennzeichnet, dass ein Timer (111) auf dem Zwischenboden (5) angeordnet ist und über einen Elektromagneten (112) und eine durch den Elektromagneten (112) bewegbare Wippe (115) mit der Gewindestange (14) in Wirkverbindung steht.

4. Schmierstoffgeber nach Anspruch 3, dadurch gekennzeichnet, dass eine Wippe (115) durch den Elektromagneten (112) in zwei Ruhestellungen bringbar und darin durch eine Feder (114) gehalten ist.

5. Schmierstoffgeber nach Anspruch 3, dadurch gekennzeichnet, dass die Wippe (115) zwei gezahnte Enden (113'') aufweist, von denen in jeder Ruhestellung jeweils eines mit einem gezahnten Rad (116), welches fest mit der Gewindestange (14) verbunden ist, im Eingriff ist.

6. Schmierstoffgeber nach Anspruch 3, dadurch gekennzeichnet, dass zwischen Deckel (1) und Zwischenboden (5) Mittel zur Bestimmung der Laufzeit angeordnet sind.

7. Schmierstoffgeber nach Anspruch 3, dadurch gekennzeichnet, dass der Timer (111) mittels eines Potentiometers (118) auf eine bestimmbare Laufzeit einstellbar ist.

8. Schmierstoffgeber nach Anspruch 3, dadurch gekennzeichnet, dass Batterien (115) zur Speisung des Timers (111) und des Elektromagneten (112) auf dem Zwischenboden (5) angeordnet sind.

9. Schmierstoffgeber nach einem der Ansprüche 1 bis 8, dadurch gekennzeichnet, dass die Gewindestange 14 mittels einer lösbaren Sperre mit dem gezahnten Rad verbunden ist, wobei die lösbare Sperre während einem Nachfüllvorgang des Vorratsraumes gelöst ist.
